(19) Europäisches Patentamt
European Patent Office
Office européen des brevets

(11) **EP 4 154 944 A1**

(12) **EUROPEAN PATENT APPLICATION**
published in accordance with Art. 153(4) EPC

(43) Date of publication:
**29.03.2023 Bulletin 2023/13**

(21) Application number: **21809315.1**

(22) Date of filing: **14.05.2021**

(51) International Patent Classification (IPC):
*A61Q 9/00* [(2006.01)]   *A61K 9/107* [(2006.01)]
*A23L 5/00* [(2016.01)]   *A61K 8/06* [(2006.01)]
*A61K 8/34* [(2006.01)]   *A61K 8/73* [(2006.01)]
*A61K 8/92* [(2006.01)]   *A61K 47/10* [(2006.01)]
*A61K 47/38* [(2006.01)]   *A61K 47/44* [(2006.01)]

(52) Cooperative Patent Classification (CPC):
**A23L 5/00; A61K 8/06; A61K 8/34; A61K 8/73;
A61K 8/92; A61K 9/107; A61K 47/10; A61K 47/38;
A61K 47/44; A61Q 19/00**

(86) International application number:
**PCT/JP2021/018428**

(87) International publication number:
**WO 2021/235352 (25.11.2021 Gazette 2021/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.05.2020   JP 2020088329**

(71) Applicant: **Daicel Corporation**
**Osaka 530-0011 (JP)**

(72) Inventors:
• **SAKAMOTO, Yuta**
  **Tokyo 108-8230 (JP)**
• **SAKANISHI, Yuichi**
  **Tokyo 108-8230 (JP)**
• **KOBAYASHI, Keiko**
  **Tokyo 108-8230 (JP)**
• **OMURA, Masaya**
  **Tokyo 108-8230 (JP)**

(74) Representative: **Grünecker Patent- und
Rechtsanwälte
PartG mbB
Leopoldstraße 4
80802 München (DE)**

(54) **EMULSIFIABLE PREPARATION, AQUEOUS COSMETIC, FOOD OR BEVERAGE AND PHARMACEUTICAL COMPOSITION**

(57)   Provided are an emulsifiable preparation that has little influence on the environment or the human body, that is excellent in long-term storage stability, and that imparts a pleasant touch feeling to the skin, and an aqueous cosmetic that contains the emulsifiable preparation, a food or beverage that contains the emulsifiable preparation, and a pharmaceutical composition that contains the emulsifiable preparation. The emulsifiable preparation includes: one or more aqueous components selected from the group consisting of water and alcohol; an oily component; and microparticles of a polymer compound, in which the microparticles contain cellulose acetate as the polymer compound, and the microparticles has an average particle size of 2 to 10 $\mu$m. The aqueous cosmetic contains the emulsifiable preparation. The food or beverage contains the emulsifiable preparation. The pharmaceutical composition contains the emulsifiable preparation.

EP 4 154 944 A1

**Description**

Technical Field

The present disclosure provides an emulsifiable preparation that has little influence on the environment or the human body, that is excellent in long-term storage stability, and that imparts a pleasant touch feeling to the skin, an aqueous cosmetic that contains the emulsifiable preparation, a food or beverage that contains the emulsifiable preparation, and a pharmaceutical composition that contains the emulsifiable preparation. The present application claims priority from the Japanese Patent Application No. 2020-088329, filed in Japan on May 20, 2020, the content of which is incorporated herein by reference.

Background Art

In recent years, an emulsification method using a surfactant or solid particles has been known. The surfactant is a general term for a compound having a hydrophilic site and a hydrophobic site in the molecule. Surfactants are used as base materials for stably mixing an aqueous phase component and an oil phase component. Surfactants are used in the production of, for example, detergents, milky lotions, and lotions. The amounts of surfactants to be produced are increasing with the development of the petrochemical industry, and it is said that the amounts reached 14.09 million tons in 2017 (Non-Patent Literature 1).

A Pickering emulsion has been proposed as the emulsification method using solid particles. The Pickering emulsion is an emulsion stabilized by solid particles adsorbed to the liquid-liquid interface. As solid particles that form a Pickering emulsion, for example, various powder particles of inorganic particles (hydrophobic silica, clay mineral, iron oxide, and carbon black), and organic particles (polymer latex, and the like) are used (Non-Patent Literature 2). In addition, a Pickering emulsion using solid particles containing an acrylic polymer has been proposed. (Patent Document 1) Furthermore, an emulsification method using nano-sized fine particles has also been reported (Patent Document 2).

The surfactant has had safety issues for a long time (Non-Patent Literatures 3 and 4). A typical surfactant is, for example, sodium lauryl sulfate. It is known that sodium lauryl sulfate binds to a hydrophobic part of a protein and brings the charge of a hydrophilic part of sodium lauryl sulfate onto the surface of the protein, thereby causing protein denaturation (Non-Patent Literature 5).

Citation List

Patent Document

Non-Patent Literature 1: The Future of Surfactants to 2022 (Smithers Apex, 2017)
Non-Patent Literature 2: Colloids and Surfaces A: Physicochemical and Engineering Aspects, Volume 439, December 20, 2013, pp. 23-34
Non-Patent Literature 3: DETERGENT & COSMETICS p. 28, Vol. 31, No. 1, Jan. 2008
Non-Patent Literature 4: DETERGENT & COSMETICS p. 11, Vol. 25, No. 4, Aug. 2002
Non-Patent Literature 5: The bulletin of Okayama University of Science: Number 50, Appl 3-22 (2014)

Patent Document 1: JP 2019-202962 A
Patent Document 2: JP 2009-161460 A

Summary of Invention

Technical Problem

When solid particles are used in the emulsification method, storage stability of the emulsifiable preparation may not be sufficient. Further, the use of inorganic particles as solid particles causes the touch feeling to the skin to deteriorate. When organic particles are used as solid particles, marine contamination by microplastics is concerned. Thus, an emulsification method using nanoparticles having biodegradability as solid particles is also devised. However, there is a concern about the potential risk of nanoparticles themselves. Furthermore, when an emulsifiable preparation containing nanoparticles is applied to the skin, the nanoparticles are caught in the wrinkles on the skin, and thus the touch feeling to the skin after application is not pleasant. Consequently, emulsifiable preparations containing solid particles have not been widely used in cosmetics and the like so far.

Therefore, an object of the present disclosure is to provide an emulsifiable preparation that has little influence on the environment or the human body, that is excellent in long-term storage stability, and that imparts a pleasant touch feeling to the skin, an aqueous cosmetic that contains the emulsifiable preparation, a food or beverage that contains the emulsifiable preparation, and a pharmaceutical composition that contains the emulsifiable preparation.

Solution to Problem

As a result of diligent research to achieve the above-described object, the present inventors have found that the use of specific microparticles in the emulsification method enables an emulsifiable preparation that has little influence on the environment or the human body, that is excellent in long-term storage stability, and that imparts a pleasant touch feeling to the skin. The present disclosure has been completed based on these findings.

That is, the present disclosure provides an emulsifiable preparation including: one or more aqueous components selected from the group consisting of water and alcohol; an oily component; and microparticles of a polymer compound, in which the microparticles contain cellulose acetate as the polymer compound, and the microparticles has an average particle size of 2 to 10 $\mu$m.

The alcohol may contain a polyhydric alcohol.

The amount of the polyhydric alcohol may be 20 wt.% or greater relative to the total amount of the alcohol.

The emulsifiable preparation may further contain a thickener.

The emulsifiable preparation may further contain a surfactant.

The present disclosure provides an aqueous cosmetic, a food or beverage, or a pharmaceutical composition, each of which includes the emulsifiable preparation described above.

Advantageous Effects of Invention

According to the emulsifiable preparation of the present disclosure, the cellulose acetate contained in the microparticles is biodegradable. Thus, the microparticles can reduce the influence on the environment or the human body. Further, the microparticles have an average particle size of 2 to 10 $\mu$m. Accordingly, it is possible to produce an emulsifiable preparation that can reduce the potential risk of nanoparticles themselves, that is excellent in long-term storage stability, and that imparts a pleasant touch feeling to the skin. In addition, the emulsifiable preparation of the present disclosure can be suitably used as an aqueous cosmetic, a food or beverage, or a pharmaceutical composition.

Description of Embodiments

Hereinafter, embodiments for carrying out the present disclosure will be described. Note that each of the configurations, combinations thereof, and the like in each of the embodiments are an example, and various additions, omissions, substitutions, and other changes may be made as appropriate without departing from the spirit of the present disclosure. The present disclosure is not limited by the embodiments and is limited only by the claims.

Emulsifiable Preparation

The emulsifiable preparation of the present disclosure (hereinafter, sometimes referred to as "emulsifiable preparation") is a Pickering emulsion. The emulsifiable preparation is produced by an emulsification method using microparticles as solid particles. The emulsifiable preparation includes an aqueous component, an oily component, and microparticles. The microparticles adsorb to the emulsifying interface between the aqueous component and the oily component. The oily component in a state of being covered with the microparticles is dispersed in the aqueous component. This makes it possible to stably maintain a state in which the oily component is uniformly dispersed in the aqueous component. Therefore, an emulsifiable preparation being in excellent storage stability is produced. Hereinafter, the microparticles, the aqueous component, and the oily component will be described in detail.

Microparticles

In the emulsifiable preparation of the present disclosure, microparticles of a polymer compound contain cellulose acetate as the polymer compound. Since cellulose acetate is a polymer compound, cellulose acetate is hardly absorbed from the skin and intestinal wall, and is safe for the human body. From a long-time perspective, cellulose acetate, i.e., a biodegradable polymer compound, is decomposed even when it is released in nature, and thus the influence on the environment can be suppressed.

The content of the polymer compound contained in the microparticles is preferably 60 wt.% or greater, more preferably 80 wt.% or greater, and still more preferably 95 wt.% or greater relative to the total weight of the microparticles.

When the content of the polymer compound contained in the microparticles is 60 wt.% or greater relative to the total weight of the microparticles, microparticles are more likely to have a well-ordered shape.

In addition, the microparticles are not limited to cellulose acetate, and may contain one or more other polymer compounds. The microparticles may be, for example, particles of cellulose acetate coated with another polymer compound, may be particles of another polymer compound coated with cellulose acetate, or may be particles formed of a mixture of cellulose acetate and another polymer compound. When the microparticles contain another polymer compound, the microparticles can have properties that are not possessed by cellulose acetate. Thus, for example, the taste, color, and feeling to the touch of the emulsifiable preparation can vary depending on the intended use.

The content of cellulose acetate contained in the polymer compound of the microparticles is preferably 40 wt.% or greater, more preferably 60 wt.% or greater, and still more preferably 80 wt.% or greater relative to the total weight of the polymer compound.

Another polymer compound contained in the microparticles is preferably a biodegradable polymer compound. Examples of the biodegradable polymer compound include polylactic acid, polyglycolic acid, polyaspartic acid, polyvinyl alcohol, polyhydroxyalkanoate, modified polyethylene terephthalate, starch (glucose polymer), cellulose derivatives other than cellulose acetate, polybutylene succinate-based compounds, polycaprolactone, and gelatin. When the emulsifiable preparation is used for foods or beverages, another polymer compound may be, for example, an edible polysaccharide. Examples of the polysaccharide include pullulan, gellan gum, xanthan gum, tamarind seed gum, locust bean gum, pectin, carrageenan, guar gum, gum arabic, dextran, dextrin, sodium chondroitin sulfate, sodium hyaluronate, and sodium alginate. When the emulsifiable preparation is used for cosmetics, examples of another polymer compound include polyvinylpyrrolidone, carboxyvinyl polymer, sodium polyacrylate, methacrylic acid copolymer, and polyethylene glycol.

The microparticles have an average particle size of 2 to 10 $\mu$m. The upper limit of the average particle size of the microparticles is preferably 8 $\mu$m, and more preferably 7 $\mu$m. The lower limit of the average particle size of the microparticles is preferably 4 $\mu$m, and more preferably 5 $\mu$m. The microparticles have an average particle size of 2 to 10 $\mu$m, and thus the microparticles are not caught in wrinkles on the skin when the emulsifiable preparation is applied to the skin, and the touch feeling to the skin after application is improved. In addition, when the microparticles have an average particle size of 2 to 10 $\mu$m, the storage stability of the emulsifiable preparation is improved. Furthermore, when the average particle size of the microparticles is 5 $\mu$m or greater, the microparticles can impart a pleasant touch feeling to the emulsifiable preparation due to a ball-bearing effect.

The average particle size can be measured using dynamic light scattering. The specific measurement procedure is as follows. First, the microparticles are suspended at a concentration of 100 ppm in pure water using an ultrasonic vibrating apparatus to prepare a sample. Then, the average particle size can be derived from the particle size volume distribution measured by laser diffraction ("Laser Diffraction/Scattering Particle Size Distribution Measuring Apparatus LA-960" available from Horiba Ltd., ultrasonic treatment for 15 minutes, and a refractive index (1.500, medium (water; 1.333)). The average particle size (such as in nm and $\mu$m) herein refers to the value of the particle size corresponding to 50% of the integrated scattering intensity in this particle size distribution.

Microparticles containing cellulose acetate as a polymer compound (hereinafter, sometimes referred to as "cellulose acetate microparticles") can be prepared by the following method (see WO 2019/156116 A1). A method for producing cellulose acetate microparticles includes: preparing cellulose acetate impregnated with a plasticizer; preparing a dispersion containing, as a dispersoid, the cellulose acetate impregnated with the plasticizer; and removing a water-soluble polymer from the prepared dispersion. Note that the cellulose acetate microparticles are not limited to being prepared by the following method, and may be prepared by another known method.

The coefficient of variation of the particle size of the cellulose acetate microparticles of the present disclosure is preferably 0% or greater and 60% or less, and more preferably 2% or greater and 50% or less. The coefficient of variation (%) of the particle size can be calculated by an equation: (standard deviation of particle size)/(average particle size) $\times$ 100. When the coefficient of variation of the particle size of the cellulose acetate microparticles is 0% or greater and 60% or less, the particle size of the cellulose acetate microparticles is within a certain range. Therefore, when the cellulose acetate microparticles are used in the aqueous cosmetic material, the feeling to the touch of the aqueous cosmetic material is improved.

The sphericity of the cellulose acetate microparticles of the present disclosure is 0.7 or greater and 1.0 or less, preferably 0.8 or greater and 1.0 or less, and more preferably 0.9 or greater and 1.0 or less. When the sphericity of the cellulose acetate microparticles is 0.7 or greater and 1.0 or less, the feeling to the touch of the microparticles is excellent. Therefore, even when the cellulose acetate microparticles are used in the aqueous cosmetic material, the feeling to the touch of the aqueous cosmetic material and the soft focus effect are improved.

The sphericity can be measured by the following method. 30 particles are randomly selected from an image of cellulose acetate microparticles observed with a scanning electron microscope (SEM), the major axis length and the minor axis length of the selected particles are measured, and the ratio (minor axis length)/(major axis length) of each particle is determined. Then, the average value of the ratios (minor axis length)/(major axis length) is defined as the sphericity. The closer to 1 the sphericity is, the closer to the true sphere the particles can be determined to be.

Preparation of Cellulose Acetate Impregnated with Plasticizer

The cellulose acetate impregnated with the plasticizer is prepared by mixing the cellulose acetate and the plasticizer. The total degree of acetyl substitution of cellulose acetate is preferably 0.7 or greater and 2.9 or less, more preferably 1.4 or greater and less than 2.6, and still more preferably 2.0 or greater and less than 2.6. When the total degree of acetyl substitution is 0.7 or greater and 2.9 or less, cellulose acetate is less soluble in water, and thus the sphericity of the particles to be produced increases. The particles also have sufficiently high biodegradability. The cellulose acetate having a total degree of acetyl substitution of 0.7 or greater and 2.9 or less is produced by a known method for producing cellulose acetate. Examples of such a known method for producing cellulose acetate include a so-called acetic acid method in which acetic anhydride is used as an acetylating agent, acetic acid is used as a diluent, and sulfuric acid is used as a catalyst.

The total degree of acetyl substitution of the cellulose acetate can be measured by the following method. First, the total degree of acetyl substitution is the sum of the degrees of acetyl substitution at the 2-, 3-, and 6-positions of the glucose ring of cellulose acetate. The degrees of acetyl substitution at the 2-, 3-, and 6-positions of the glucose ring of cellulose acetate particles can be measured by NMR according to the method of Tezuka (Tezuka, Carbonydr. Res. 273, 83 (1995)). Specifically, free hydroxyl groups of a cellulose acetate sample are propionylated with propionic anhydride in pyridine. The resulting sample is dissolved in deuterated chloroform, and the $^{13}$C-NMR spectrum is measured. The carbon signals of the acetyl group appear in the region from 169 ppm to 171 ppm in the order of 2-, 3-, and 6-positions from the high magnetic field; and the carbonyl carbon signals of the propionyl group appear in the region from 172 ppm to 174 ppm in the same order. The degrees of acetyl substitution at the 2-, 3-, and 6-positions of the glucose ring in the original cellulose acetate can be determined based on the presence ratio of the acetyl group and the propionyl group at the respective corresponding positions. The degree of acetyl substitution can also be analyzed by $^{1}$H-NMR in addition to $^{13}$C-NMR.

Furthermore, the total degree of acetyl substitution is determined by converting the acetylation degree determined according to the method for measuring the acetylation degree in ASTM: D-817-91 (Testing methods for cellulose acetate, etc.). This is the most common procedure to determine the degree of substitution of cellulose acetate.

$$DS = 162.14 \times AV \times 0.01/(60.052 - 42.037 \times AV \times 0.01)$$

In the above equation, DS is the total degree of acetyl substitution, and AV is the acetylation degree (%). Note that the value of the degree of substitution calculated by the conversion usually has a slight discrepancy from the value measured by NMR described above. When the converted value and the value measured by NMR are different, the value measured by NMR is adopted. In addition, if the value varies among the specific methods of NMR measurement, the value measured by NMR according to the method of Tezuka described above is adopted.

The method for measuring the acetylation degree according to ASTM: D-817-91 (Testing methods for cellulose acetate, etc.) is outlined as follows. First, 1.9 g of dried cellulose acetate is accurately weighed and dissolved in 150 mL of a mixed solution of acetone and dimethylsulfoxide (a volume ratio of 4:1), then 30 mL of a 1 N sodium hydroxide solution is added, and the cellulose acetate is saponified at 25°C for 2 hours. Phenolphthalein is added as an indicator, and the excess sodium hydroxide is titrated with IN-sulfuric acid (concentration factor: F). In addition, a blank test is performed in the same manner as described above, and the acetylation degree is calculated according to the following equation.

$$\text{Average acetylation degree (\%)} = \{6.5 \times (B - A) \times F\}/W$$

where A represents the titration volume (mL) of the 1 N sulfuric acid for the sample, B represents the titration volume (mL) of the 1 N sulfuric acid for the blank test, F represents the concentration factor of the 1 N sulfuric acid, and W represents the weight of the sample.

The plasticizer can be used without any particular limitation as long as it has a plastic effect in the process of melting and extruding cellulose acetate. For example, the plasticizer is preferably at least one selected from the group consisting of citrate-based plasticizers containing a citrate ester, such as triethyl citrate, acetyl triethyl citrate, and acetyl tributyl citrate; glycerin ester-based plasticizers containing a glycerin alkyl ester, such as triacetin, diacetin, and monoacetin; adipate-based plasticizers, such as diisononyl adipate; and phthalate-based plasticizers, such as ethyl phthalate and methyl phthalate; more preferably at least one selected from the group consisting of triethyl citrate, acetyl triethyl citrate, acetyl tributyl citrate, triacetin, and diisononyl adipate; and even more preferably at least one selected from the group consisting of acetyl triethyl citrate, triacetin, diacetin, and diethyl phthalate. The single plasticizer can be used alone, or the two or more plasticizers can be used in combination.

The blending amount of the plasticizer is preferably more than 0 part by weight and 40 parts by weight or less, more preferably 2 parts by weight or greater and 40 parts by weight or less, still more preferably 10 parts by weight or greater and 30 parts by weight or less, and most preferably 15 parts by weight or greater and 20 parts by weight or less relative to 100 parts by weight of the total amount of the cellulose acetate and the plasticizer. When the plasticizer is blended in an amount of more than 0 part by weight and 40 parts by weight or less relative to 100 parts by weight of the total amount of the cellulose acetate and the plasticizer, the sphericity of the cellulose acetate microparticles to be produced increases.

The cellulose acetate and the plasticizer can be dry-mixed or wet-mixed using an existing mixer, such as a Henschel mixer. When a mixer is used, the temperature inside the mixer is preferably in a range of 20°C or higher and less than 200°C. When the temperature inside the mixer is 20°C or higher and less than 200°C, the melting of the cellulose acetate can be prevented. In addition, the cellulose acetate and the plasticizer may be mixed by melt-kneading. The melt-kneading may be performed in combination with mixing using a mixer, and in this case, the melt-kneading is preferably performed after mixing in temperature conditions in a range of 20°C or higher and lower than 200°C using a mixer. This allows the plasticizer and cellulose acetate to be mixed more uniformly in a short period of time. Therefore, the sphericity of the cellulose acetate microparticles to be finally prepared increases, and the tactile sense and feeling to the touch of microparticles are improved.

The melt-kneading is performed by heating and mixing with an extruder. Preferably, the kneading temperature (cylinder temperature) of the extruder is in a range of 200°C to 230°C. The melting point of cellulose acetate is approximately in a range of 230°C to 280°C, although it varies depending on the degree of substitution. Here, the plasticization temperature of the cellulose acetate impregnated with the plasticizer is reduced. Thus, even when the kneading temperature of the extruder is in a range of 200°C to 230°C, it is possible to obtain a uniform kneaded product in which the cellulose acetate is plasticized. Note that the kneading temperature may be, for example, 200°C in using a twin-screw extruder. The kneaded product may be extruded in a strand shape and formed into a pellet form by hot cutting or the like. The die temperature in this case may be, for example, approximately 220°C.

Preparation of Dispersion

In preparing the dispersion, the cellulose acetate impregnated with the plasticizer and a water-soluble polymer are first kneaded. The blending amount of the water-soluble polymer is preferably 55 parts by weight or greater and 99 parts by weight or less, more preferably 60 parts by weight or greater and 90 parts by weight or less, and still more preferably 65 parts by weight or greater and 85 parts by weight or less relative to 100 parts by weight of the total amount of the cellulose acetate impregnated with the plasticizer and the water-soluble polymer.

The water-soluble polymer used in preparing a dispersion refers to a polymer having an insoluble content of less than 50 wt.% when 1 g of the polymer is dissolved in 100 g of water at 25°C. Examples of the water-soluble polymer may include polyvinyl alcohol, polyethylene glycol, sodium polyacrylate, polyvinylpyrrolidone, polypropylene oxide, polyglycerin, polyethylene oxide, vinyl acetate, modified starch, thermoplastic starch, methyl cellulose, ethyl cellulose, hydroxyethyl cellulose, and hydroxypropyl cellulose. Among them, polyvinyl alcohol, polyethylene glycol, and thermoplastic starch are preferred, and polyvinyl alcohol and thermoplastic starch are particularly preferred. Further, the thermoplastic starch can be prepared by a well-known method.

The kneading of the cellulose acetate impregnated with the plasticizer and the water-soluble polymer can be performed, for example, with an extruder such as a twin-screw extruder. The cellulose acetate impregnated with the plasticizer and the water-soluble polymer are kneaded at 200°C or higher and 280°C or lower. The temperature of the kneading refers to the cylinder temperature of the extruder. The kneaded product is extruded into a string shape from the die attached to the tip of the extruder. At this time, the die temperature may be not lower than 220°C and not higher than 300°C. The kneaded product extruded into a string shape is cut and formed into a pellet form to prepare a dispersion. The resulting dispersion is a dispersion containing: the water-soluble polymer as a dispersion medium; and the cellulose acetate impregnated with the plasticizer as a dispersoid.

Removal of Water-soluble Polymer

The removal of the water-soluble polymer from the dispersion is described. The method of removing the water-soluble polymer is not particularly limited as long as the water-soluble polymer dissolves and can be removed from the particles. Examples of the method of removing the water-soluble polymer include a method of dissolving and removing the water-soluble polymer of the dispersion using a solvent for removal of the water-soluble polymer, such as water, an alcohol, such as methanol, ethanol, or isopropanol, or their mixed solution. Specifically, examples include a method of removing the water-soluble polymer from the dispersion, such as by mixing the dispersion and the solvent for removal of the water-soluble polymer and filtering the mixture to take out the filtrate.

The mixing ratio of the dispersion and the solvent for removal of the water-soluble polymer is preferably 0.01 wt.% or greater and 20 wt.% or less, more preferably 2 wt.% or greater and 15 wt.% or less, and still more preferably 4 wt.% or greater and 13 wt.% or less relative to the total weight of the dispersion and the solvent for removal of the water-soluble polymer. When the content of the dispersion is 0.01 wt.% or greater and 20 wt.% or less relative to the total weight of the dispersion and the solvent for removal of the water-soluble polymer, the water-soluble polymer is sufficiently dissolved in the solvent, and the water-soluble polymer can be sufficiently washed and removed. In addition, it is possible to easily separate the cellulose acetate microparticles that are not dissolved in the solvent for removal of the water-soluble polymer and the water-soluble polymer that is dissolved in the solvent for removal of the water-soluble polymer, by an operation such as filtration or centrifugation.

The mixing temperature of the dispersion and the solvent for removal of the water-soluble polymer is preferably 0°C or higher and 200°C or lower, more preferably 20°C or higher and 110°C or lower, and still more preferably 40°C or higher and 80°C or lower. When the mixing temperature of the dispersion and the solvent for removal of the water-soluble polymer is 0°C or higher and 200°C or lower, the water-soluble polymer is sufficiently dissolved in the solvent for removal of the water-soluble polymer, and the water-soluble polymer can be sufficiently washed and removed. Further, the particles can be taken out while maintaining a desired shape of the particles without causing deformation, aggregation, or the like of the particles.

The mixing time of the dispersion and the solvent for removal of the water-soluble polymer is not particularly limited and may be appropriately adjusted, but may be, for example, 0.5 hours or more, 1 hour or more, 3 hours or more, or 5 hours or more, and may be 6 hours or less.

In addition, the method of mixing the dispersion and the solvent for removal of the water-soluble polymer is not limited as long as the water-soluble polymer can be dissolved, but, for example, a stirring device, such as an ultrasonic homogenizer or a Three-One Motor, can efficiently remove the water-soluble polymer from the dispersion even at room temperature. For example, when a Three-One Motor is used as the stirring device, the rotation speed during mixing the dispersion and the solvent may be, for example, not less than 5 rpm and not greater than 3000 rpm. This can more efficiently remove the water-soluble polymer from the dispersion. In addition, this also efficiently removes the plasticizer from the dispersion. This results in microparticles containing cellulose acetate (cellulose acetate microparticles).

In removing the water-soluble polymer from the dispersion, the plasticizer may be or need not be removed from the dispersion together with the water-soluble polymer. Thus, the resulting cellulose acetate microparticles may contain or need not contain a plasticizer.

Aqueous Component

In the emulsifiable preparation of the present disclosure, the aqueous component contains one or more aqueous components selected from the group consisting of water and alcohols. In the present specification, the aqueous component refers to water or a water-soluble component. Since the emulsifiable preparation contains the aqueous component, the emulsifiable preparation of the present disclosure provides a refreshing feeling to the touch.

The alcohol contained in the aqueous component is not particularly limited, but is preferably an alcohol that is highly safe to the human body. As the alcohol, for example, an alcohol that can be used as an oral preparation or an alcohol that is safe when applied to the skin can be adopted. One type of alcohol may be contained, or two or more types thereof may be contained. The type and content of the alcohol are appropriately selected according to the use, the solubility of the additive, or the like.

Examples of the alcohol include lower alcohols having from 1 to 4 carbons, such as ethanol or isopropyl alcohol, or polyhydric alcohols. Examples of the polyhydric alcohols include propylene glycol, dipropylene glycol, glycerin, diglycerin, low-molecular-weight (e.g., a weight-average molecular weight of 1000 or greater and 20000 or less) polyethylene glycol, 1,3-butylene glycol, and 1,2-pentanediol. Polyhydric alcohols have low volatility and are used as lubricants. Thus, when the aqueous component contains a polyhydric alcohol, the feeling to the touch of the emulsifiable preparation can be made smoother. The aqueous component may contain, as a polyhydric alcohol, sorbitol, xylitol, erythritol, mannose, or trehalose, each of which is individual at ordinary temperature. Such polyhydric alcohols can impart sweetness and the like to the emulsifiable preparation because they have sweetness and the like.

A proportion of the polyhydric alcohol relative to the total amount of the alcohol is preferably 20 wt.% or greater, more preferably 50 wt.% or greater, and still more preferably 100 wt.%. When the proportion of the polyhydric alcohol relative to the total amount of the alcohol is 20 wt.% or greater, the feeling to the touch of the emulsifiable preparation can be made smoother.

When the aqueous component contains water, the content of water relative to the total weight of the emulsifiable preparation is preferably 10 wt.% or less, more preferably 8 wt.% or less, and still more preferably 5 wt.% or less. When the content of water relative to the total weight of the emulsifiable preparation is more than 10 wt.%, water may decompose the biodegradable polymer compound contained in the cellulose acetate microparticles. In contrast, when the content of water relative to the total weight of the emulsifiable preparation is 10 wt.% or less, a water-soluble component such as salt becomes soluble in the emulsifiable preparation, and it is also possible to suppress the influence of water on the biodegradable polymer compound contained in the cellulose acetate microparticles.

When the aqueous component contains an alcohol, the biodegradable polymer compound contained in the cellulose acetate microparticles can suppress the influence of the aqueous component, compared with the case where the aqueous component is only water. When the aqueous component contains an alcohol, the content of the aqueous component including the alcohol is preferably 90 wt.% or less, more preferably 70 wt.% or less, and still more preferably 60 wt.% or less relative to the total weight of the emulsifiable preparation.

Oily Component

In the emulsifiable preparation of the present disclosure, the oily component refers to a component which is insoluble or poorly soluble in water. The oily component is a useful component that is oil-based and intended to be added to an aqueous cosmetic, a food or beverage, a pharmaceutical composition, or the like. The oily component is not particularly limited, but is preferably an oily component that is highly safe to the human body. As the oily component, for example, an oily component that can be used as an oral preparation or an oily component that is safe when applied to skin can be adopted. Examples of the oily component include oils and fats such as monoglyceride in which one fatty acid is bonded to glycerin, diglyceride in which two fatty acids are bonded to glycerin, and triglyceride in which three fatty acids are bonded to glycerin. Specific examples of the oils and fats include vegetable oils such as rapeseed oil, sesame oil, olive oil, coconut oil, camellia oil, corn oil, avocado oil, sasanqua oil, castor oil, jojoba oil, sunflower oil, and soybean oil.

In addition, examples of the oily component include esters, silicones, lactones, aldehydes, ketones, higher fatty acids, higher alcohols, essential oils, antioxidants, fat-soluble vitamins, and plant sterols. Examples of the esters include monoesters such as cetyl 2-ethylhexanoate, isopropyl myristate, cetyl octanoate, octyl palmitate, isocetyl stearate, isopropyl isostearate, octyl isopalmitate, and isopropyl sebacate; diesters such as diethyl sebacate, diisopropyl sebacate, di-2-ethylhexyl sebacate, and diisopropyl phthalate; and triesters such as glyceryl tri-2-ethylhexanoate and caprylic/capric triglyceride. Examples of the silicones include straight silicone oils such as dimethyl silicone oil, methyl phenyl silicone oil, and methyl hydrogen silicone oil, and modified silicone oils in which an organic group is introduced into a side chain or an end of the straight silicone oil. Examples of the lactones include gluconolactone, mevalonolactone, lactobionolactone, and pantolactone. Examples of the aldehydes include cinnamaldehyde and cinnamic aldehyde. Examples of the ketones include fragrances such as $\alpha$-diketone.

One type of oily component may be contained, or two or more types thereof may be contained. The type and content of the oily component are appropriately selected according to the use, the solubility of the additive, or the like. The oily component is preferably from about 0.1 to about $10^3$ parts by weight, more preferably from about 0.5 to about $10^2$ parts by weight, and still more preferably from about 1 to about $2 \times 10^2$ parts by weight, relative to 100 parts by weight of the cellulose acetate microparticles. When the content of the oily component is from about 0.1 to about $10^3$ parts by weight relative to 100 parts by weight of the cellulose acetate microparticles, an emulsifiable preparation stabilized by the cellulose acetate microparticles is prepared.

The emulsifiable preparation of the present disclosure may contain other components as long as the effects of the present disclosure are not impaired. For example, a surfactant, a thickener, a stabilizer, an antioxidant, a chelator, a preservative, a pH adjuster, a buffer, a flavoring substance, and a sweetening agent can be added to the emulsifiable preparation.

Examples of the surfactant include fatty acid soaps such as sodium laurate and sodium palmitate; anionic surfactants such as potassium lauryl sulfate, and triethanolamine alkyl sulfate ether; cationic surfactants such as stearyltrimethylammonium chloride, benzalkonium chloride, and laurylamine oxide; imidazoline-based amphoteric surfactants such as 2-cocoyl-2-imidazolinium hydroxide-1-carboxyethyloxy 2 sodium salt; betaine-based surfactants such as alkyl betaine, amide betaine, and sulfobetaine; amphoteric surfactants such as acyl methyl taurine; sorbitan fatty acid esters such as sorbitan monostearate and sorbitan sesquioleate; glycerin fatty acid esters such as glycerin monostearate; propylene glycol fatty acid esters such as propylene glycol monostearate; hydrogenated castor oil derivatives; glycerin alkyl ethers; polyoxyethylene sorbitan fatty acid esters; polyoxyethylene sorbitol fatty acid esters; polyoxyethylene glycerin fatty acid esters; polyoxyethylene fatty acid esters; polyoxyethylene alkyl ethers; polyoxyethylene alkyl phenyl ethers; Pluronic (registered trademark) types; polyoxyethylene/polyoxypropylene alkyl ethers; Tetronic types; polyoxyethylene castor oil/hydrogenated castor oil derivatives; nonionic surfactants such as sucrose fatty acid esters and alkyl glucosides; sodium dodecyl sulfate; vitamin E derivatives; and phospholipids.

The amount of the surfactant is preferably from 0.01 to 100 parts by weight, more preferably from 0.1 to 80 parts by weight, and still more preferably from 1 to 50 parts by weight relative to 100 parts by weight of the polymer compound (e.g., cellulose acetate) contained in the cellulose acetate microparticles. When the amount of the surfactant is from 0.01 to 100 parts by weight relative to 100 parts by weight of the polymer compound contained in the cellulose acetate microparticles, the emulsifiable preparation can further improve the dispersibility of the oily component.

Examples of the thickener include xanthan gum, curdlan, pullulan, guar gum derivatives, locust bean gum, carrageenan, pectin, cellulose derivatives such as hydroxyethyl cellulose and carboxymethyl cellulose, carbomers (carboxyvinyl polymers), pectin, β-glucan, tamarind gum, polyvinylpyrrolidone, polyvinyl alcohol, polyacrylic acid, alginic acid, hyaluronic acid, and polyalkylene glycol, and salts thereof. One kind of these thickeners can be used alone or two or more kinds thereof can be used in combination. The thickener is preferably a carboxyvinyl polymer in terms of having low irritation and a high thickening effect, having low changes over time in viscosity, and having strong resistance to contamination by microorganisms. The emulsifiable preparation contains a thickener, and thus the dispersibility of the oily component and the cellulose acetate microparticles can be further stabilized.

The emulsifiable preparation of the present disclosure can be prepared, for example, by the following method. Cellulose acetate microparticles are stirred together with an aqueous component for a predetermined time. As a result, the cellulose acetate microparticles are dispersed in the aqueous component to get wet. An oily component is added to the wet cellulose acetate microparticles, and the mixture is stirred for a predetermined time. As a result, the oily component and the aqueous component are emulsified by the cellulose acetate microparticles, and thereby an emulsifiable preparation was yielded. The emulsifiable preparation may be prepared by another known method without being limited to the preparation method described above.

The emulsifiable preparation of the present disclosure is suitable for an external preparation for skin. The emulsifiable preparation of the present disclosure is excellent in storage stability and feeling to the touch, and thus can be used for aqueous cosmetics. An aqueous cosmetic can be prepared, for example, by the following method. In the emulsifiable preparation of the present disclosure, for example, an aqueous component such as water or other additives is/are added and the mixture is stirred for a predetermined time. As a result, an aqueous cosmetic is prepared in which the emulsifiable preparation supports the intended use.

The emulsifiable preparation of the present disclosure can be, for example, a sunscreen, a makeup base, a foundation, a lotion, a lipstick, a lip gloss, a hair care product, or the like. Cellulose acetate microparticles contained in the emulsifiable preparation of the present disclosure are safe for the human body. Accordingly, the emulsifiable preparation of the present disclosure can be suitably used for foods or beverages. The emulsifiable preparation of the present disclosure can be, for example, a cream, a sauce, a jelly, or a health food such as a supplement, or other beverages. In addition, the emulsifiable preparation of the present disclosure can be a pharmaceutical preparation (pharmaceutical composition) such as an oral preparation, an injection, or an external preparation such as an ointment or a poultice.

Examples

Hereinafter, the present disclosure will be described more specifically with reference to examples, but the present disclosure is not limited to these examples, and modifications and improvements within the scope of achieving the object of the present disclosure are encompassed by the present disclosure. Note that the microparticles used in Examples 1 to 2 and Comparative Example 2 were prepared by the method shown in Production Examples 1 to 3 below.

Production Example 1

First, 100 parts by weight of cellulose diacetate (available from Daicel Corporation: total degree of acetyl substitution DS = 2.4) and 25 parts by weight of triacetin as a plasticizer were blended in a dry state, dried at 80°C for 12 hours or more, and further stirred and mixed using a Henschel mixer to prepare a mixture of the cellulose acetate and the plasticizer. The resulting mixture was fed to a twin-screw extruder (PCM30 available from Ikegai Corp., a cylinder temperature of 200°C, a die temperature of 220°C), melt-kneaded, extruded, pelletized, and a kneaded product was formed.

Then, 32 parts by weight of the pellets of the resulting kneaded product and 68 parts by weight of polyvinyl alcohol (available from The Nippon Synthetic Chemical Industry Co., Ltd., a melting point of 190°C, a saponification degree of 99.1%) as a water-soluble polymer were blended in a dry state, then fed to a twin-screw extruder (PCM30 available from Ikegai Corp., a cylinder temperature of 220°C, a die temperature of 220°C), extruded, and a dispersion was formed.

The resulting dispersion was combined with pure water (a solvent) to give a concentration of not higher than 5 wt.% (weight of dispersion/(weight of dispersion + weight of pure water) × 100), and the mixture was stirred using a Three-One Motor (BL-3000 available from Shinto Scientific Co., Ltd.) at a rotation speed of 500 rpm, at a temperature of 80°C for 5 hours. The solution after stirring was filtered off with filter paper (No. 5A available from ADVANTEC), and the filtrate was taken out. The resulting filtrate was combined with pure water again to adjust the concentration of the dispersion to 5 wt.% or less, the mixture was further stirred at a rotation speed of 500 rpm, at a temperature of 80°C for 5 hours, and the resultant solution was filtered off to take out the filtrate. This operation was repeated three or more times, and cellulose acetate microparticles were prepared. The average particle size of the resulting cellulose acetate microparticles was measured by the following method.

The average particle size was measured using dynamic light scattering. First, the sample was combined with pure water to adjust the concentration to approximately 100 ppm, and a pure water suspension was prepared using an ultrasonic vibrating device. Then, the particle size volume distribution was determined by laser diffraction ("Laser Diffraction/Scattering Particle Size Distribution Measuring Apparatus LA-960" available from Horiba Ltd., ultrasonic treatment for 15 minutes, a refractive index (1.500, medium (water; 1.333)), and the average particle size was measured. The average particle size (in nm, μm, etc.) herein was the value of the particle size corresponding to 50% of the integrated scattering intensity in the particle size volume distribution. The result for the average particle size of the resulting cellulose acetate microparticles is shown in Table 1.

Production Example 2

A kneaded product was prepared in the same manner as in Production Example 1 except that the amount of triacetin was changed to 22 parts by weight; a dispersion was formed in the same manner as in Production Example 1 except that the amount of the pellets of the resulting kneaded product was changed to 34 parts by weight and the amount of polyvinyl alcohol was changed to 66 parts by weight; and cellulose acetate microparticles were prepared in the same manner as in Production Example 1 except that the resulting dispersion was combined with pure water to have a concentration of 5 wt.% or less, and the mixture was vigorously stirred at a rotation speed (200 rpm) at a temperature of 80°C for 5 hours. The result for the average particle size of the resulting cellulose acetate microparticles is shown in Table 1.

Production Example 3

A kneaded product was prepared in the same manner as in Production Example 1 except that triacetin was replaced with acetyl triethyl citrate as the plasticizer; a dispersion was formed in the same manner as in Production Example 1 except that the amount of the pellets of the resulting kneaded product was changed to 14 parts by weight and the amount of polyvinyl alcohol was changed to 86 parts by weight; and cellulose acetate microparticles were prepared in the same manner as in Production Example 1 except that the resulting dispersion was combined with pure water to have a concentration of 5 wt.% or less, and the mixture was vigorously stirred at a rotation speed (100 rpm) at a temperature of 80°C for 3 hours. The result for the average particle size of the resulting cellulose acetate microparticles is shown in Table 1.

Example 1

As shown in Table 1 below, 2 parts by weight of cellulose acetate microparticles (average particle size: 5 μm, available from Daicel Corporation) of Production Example 1 as microparticles, 5 parts by weight of 1,3-butylene glycol (available from Daicel Corporation) as an aqueous component, 3 parts by weight of dipropylene glycol (available from ADEKA Corporation) as an aqueous component, 5 parts by weight of cetyl 2-ethylhexanoate (available from Kokyu Alcohol Kogyo Co., Ltd.) as an oily component, 64 parts by weight of pure water as a solvent, 20 parts by weight of a 1 wt.% carbomer solution (AQUPEC 705E, available from Sumitomo Seika Chemicals Company, Limited.), and 1 part by weight of a 10 wt.% potassium hydroxide solution as a solvent were prepared.

The prepared cellulose acetate microparticles and the aqueous component were stirred at ordinary temperature for 3 minutes using a mill: Disper (available from PRIMIX Corporation), and then the resulting mixed liquid was left to stand still for 10 minutes to wet the cellulose acetate microparticles with the aqueous component. The oily component was added to the mixed liquid after wetting, and the mixture was stirred at ordinary temperature for 10 minutes using the mill: Disper (available from PRIMIX Corporation), and thereby an emulsifiable preparation was yielded. Subsequently, a solvent was added to the resulting emulsifiable preparation, and the mixture was stirred at ordinary temperature for 10 minutes using the mill: Disper (available from PRIMIX Corporation), and thereby an aqueous cosmetic was yielded.

Example 2 and Comparative Examples 1 and 2

An aqueous cosmetic was prepared in the same manner as in Example 1 except that cellulose acetate microparticles were changed as shown in Table 1 below. In Example 2, the cellulose acetate microparticles prepared in Production Example 2 were used as microparticles. In Comparative Example 1, silica particles (Sunsphere L-51, average particle size: 5 μm, available from AGC Si-Tech Co., Ltd.) were used as microparticles. Further, in Comparative Example 2, the cellulose acetate microparticles prepared in Production Example 3 were used as microparticles.

Evaluation

The dispersion stability and the touch feeling to the skin of the aqueous cosmetics prepared in Examples 1 and 2 and Comparative Examples 1 and 2 were evaluated by the following methods.

Dispersion Stability Evaluation

20 mL of the resulting aqueous cosmetic was placed in a screw bottle and left to stand still for 3 months under conditions at 25°C and 75% RH, followed by visual observation of whether or not the aqueous cosmetic was uniform. Then, the dispersion stability was evaluated in the following criteria.

Evaluation Criteria
Uniform: Good
Ununiform: Poor

Touch Feeling to the Skin

10 monitors applied 1 g of the aqueous cosmetic to their skins, and evaluated whether or not the aqueous cosmetic was smooth to the touch according to the following criteria.

Evaluation Criteria

8 or more monitors answered that the aqueous cosmetic was smooth to the touch: Good
3 to 7 monitors answered that the aqueous cosmetic was smooth to the touch: Marginal
2 or less monitors answered that the aqueous cosmetic was smooth to the touch: Poor

The results are summarized and shown in Table 1 below.

[Table 1]

Table 1

| | Component | Example 1 | Example 2 | Comparative Example 1 | Comparative Example 2 |
|---|---|---|---|---|---|
| Microparticles | Cellulose acetate microparticles (average particle size: 5 $\mu$m) | 2 | | | |
| | Cellulose acetate microparticles (average particle size: 7 $\mu$m) | | 2 | | |
| | Silica particles (average particle size: 5 $\mu$m) | | | 2 | |
| | Cellulose acetate microparticles (average particle size: 0.3 $\mu$m) | | | | 2 |
| Aqueous component | 1,3-butylene glycol | 5 | 5 | 5 | 5 |
| | Dipropylene glycol | 3 | 3 | 3 | 3 |
| Oily component | Cetyl ethylhexanoate | 5 | 5 | 5 | 5 |
| Solvent | Water | 64 | 64 | 64 | 64 |
| | 1 wt.% carbomer solution | 20 | 20 | 20 | 20 |
| | 10% KOH solution | 1 | 1 | 1 | 1 |
| Evaluation | Dispersion stability | Good | Good | Good | Good |
| | Feeling to the touch | Good | Good | Marginal | Poor |

EP 4 154 944 A1

Each aspect disclosed in the present specification can be combined with any other feature disclosed herein.
To summarize the above, configurations and variations according to embodiments of the present invention will be described below.

[1] An emulsifiable preparation including:

one or more aqueous components selected from the group consisting of water and alcohol;
an oily component; and
microparticles of a polymer compound,
in which the microparticles contain cellulose acetate as the polymer compound, and
the microparticles has an average particle size of 2 to 10 $\mu$m.

[2] The emulsifiable preparation according to [1], in which a content of the polymer compound contained in the microparticles is 60 wt.% or greater, 80 wt.% or greater, or 95 wt.% or greater relative to a total weight of the microparticles.
[3] The emulsifiable preparation according to [1] or [2], in which a content of the cellulose acetate contained in the polymer compound is 40 wt.% or greater, 60 wt.% or greater, or 80 wt.% or greater, relative to a total weight of the polymer compound.
[4] The emulsifiable preparation according to any one of [1] to [3], further including a polymer compound other than cellulose acetate (another polymer compound), in which
the other polymer compound is at least one selected from the group consisting of biodegradable polymer compounds such as polylactic acid, polyglycolic acid, polyaspartic acid, polyvinyl alcohol, polyhydroxyalkanoate, modified polyethylene terephthalate, starch (glucose polymer), cellulose derivatives other than cellulose acetate, polybutylene succinate-based compounds, polycaprolactone, and gelatin; polysaccharides such as pullulan, gellan gum, xanthan gum, tamarind seed gum, locust bean gum, pectin, carrageenan, guar gum, gum arabic, dextran, dextrin, sodium chondroitin sulfate, sodium hyaluronate, and sodium alginate; polyvinylpyrrolidone, carboxyvinyl polymer, sodium polyacrylate, methacrylic acid copolymer, and polyethylene glycol.
[5] The emulsifiable preparation according to any one of [1] to [4], in which the microparticles have an average particle size of 2 to 10 $\mu$m, or an upper limit of the average particle size is 8 $\mu$m or 7 $\mu$m, and a lower limit of the average particle size is 4 $\mu$m or 5 $\mu$m.
[6] The emulsifiable preparation according to any one of [1] to [5], in which the cellulose acetate has a total degree of acetyl substitution of 0.7 or greater and 2.9 or less, 1.4 or greater and less than 2.6, or 2.0 or greater and less than 2.6.
[7] The emulsifiable preparation according to any one of [1] to [6], in which the cellulose acetate is cellulose acetate impregnated with a plasticizer.
[8] The emulsifiable preparation according to [7], in which the plasticizer is at least one selected from the group consisting of citrate-based plasticizers containing a citrate ester, such as triethyl citrate, acetyl triethyl citrate, and acetyl tributyl citrate; glycerin ester-based plasticizers containing a glycerin alkyl ester, such as triacetin, diacetin, and monoacetin; adipate-based plasticizers, such as diisonony1 adipate; and phthalate-based plasticizers, such as ethyl phthalate and methyl phthalate.
[9] The emulsifiable preparation according to [7] or [8], in which a blending amount of the plasticizer is more than 0 parts by weight and 40 parts by weight or less, 2 parts by weight or greater and 40 parts by weight or less, 10 parts by weight or greater and 30 parts by weight or less, or 15 parts by weight or greater and 20 parts by weight or less, relative to 100 parts by weight of the total amount of the cellulose acetate and the plasticizer.
[10] The emulsifiable preparation according to any one of [1] to [9], in which the alcohol contains a polyhydric alcohol.
[11] The emulsifiable preparation according to [10], in which the polyhydric alcohol is at least one selected from the group consisting of propylene glycol, dipropylene glycol, glycerin, diglycerin, low-molecular-weight (e.g., a weight-average molecular weight of 1000 or greater and 20000 or less) polyethylene glycol, 1,3-butylene glycol, and 1,2-pentanediol.
[12] The emulsifiable preparation according to [10] or [11], in which an amount of the polyhydric alcohol is 20 wt.% or greater, 50 wt.% or greater, or 100 wt.% relative to a total amount of the alcohol.
[13] The emulsifiable preparation according to any one of [1] to [12], further including a thickener.
[14] The emulsifiable preparation according to any one of [1] to [13], further including a surfactant.
[15] An aqueous cosmetic including the emulsifiable preparation described in any one of [1] to [14].
[16] A food or beverage including the emulsifiable preparation described in any one of [1] to [14].
[17] A pharmaceutical composition including the emulsifiable preparation described in any one of [1] to [14].

# EP 4 154 944 A1

Industrial Applicability

In the emulsifiable preparation of the present disclosure, a state can be stably maintained over time in which an oily component is dispersed by cellulose acetate microparticles which are safe for the human body and the environment, and a pleasant feeling is given to the skin when applied to the skin. As a result, the emulsifiable preparation can be suitably used for aqueous cosmetics. In addition, the emulsifiable preparation can be suitably used for foods or beverages, and suitably used, particularly for health foods and the like. Furthermore, the emulsifiable preparation can be suitably used for pharmaceutical preparations such as oral preparations, injections, and external preparations.

**Claims**

1.  An emulsifiable preparation comprising:

    one or more aqueous components selected from the group consisting of water and alcohol;
    an oily component; and
    microparticles of a polymer compound,
    wherein the microparticles contain cellulose acetate as the polymer compound, and
    the microparticles has an average particle size of 2 to 10 μm.

2.  The emulsifiable preparation according to claim 1, wherein the alcohol contains a polyhydric alcohol.

3.  The emulsifiable preparation according to claim 2, wherein an amount of the polyhydric alcohol is 20 wt.% or greater relative to a total amount of the alcohol.

4.  The emulsifiable preparation according to any one of claims 1 to 3, further comprising a thickener.

5.  The emulsifiable preparation according to any one of claims 1 to 4, further comprising a surfactant.

6.  An aqueous cosmetic comprising the emulsifiable preparation of any one of claims 1 to 5.

7.  A food or beverage comprising the emulsifiable preparation of any one of claims 1 to 5.

8.  A pharmaceutical composition comprising the emulsifiable preparation of any one of claims 1 to 5.

13

**INTERNATIONAL SEARCH REPORT**

| International application No. |
| --- |
| PCT/JP2021/018428 |

A. CLASSIFICATION OF SUBJECT MATTER
A61Q 19/00(2006.01)i; A61K 9/107(2006.01)i; A23L 5/00(2016.01)i; A61K
8/06(2006.01)i; A61K 8/34(2006.01)i; A61K 8/73(2006.01)i; A61K
8/92(2006.01)i; A61K 47/10(2006.01)i; A61K 47/38(2006.01)i; A61K
47/44(2017.01)i
FI:     A61K8/34; A61K8/92; A61K8/73; A61K8/06; A61Q19/00; A61K47/10;
        A61K47/44; A61K47/38; A61K9/107; A23L5/00 K
According to International Patent Classification (IPC) or to both national classification and IPC

B. FIELDS SEARCHED

Minimum documentation searched (classification system followed by classification symbols)
A61Q19/00-90/00; A61K9/00-9/72; A23L5/00-5/30; A23L29/00-29/10; A61K8/00-
8/99; A61K47/00-47/69; A61K31/33-33/44; B01J13/00; B01J13/02-13/22

Documentation searched other than minimum documentation to the extent that such documents are included in the fields searched
        Published examined utility model applications of Japan         1922-1996
        Published unexamined utility model applications of Japan        1971-2021
        Registered utility model specifications of Japan               1996-2021
        Published registered utility model applications of Japan       1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2014-221724 A (JO COSMETICS CO., LTD.) 27 November 2014 (2014-11-27) paragraphs [0016], [0022], [0035]-[0037], [0039], [0056] | 1-6, 8 |
| X | JP 11-269049 A (YAKULT HONSHA CO., LTD.) 05 October 1999 (1999-10-05) paragraphs [0019], [0022]-[0028] | 1-6, 8 |
| X | JP 2002-241211 A (FANCL CORPORATION) 28 August 2002 (2002-08-28) claims, paragraphs [0006], [0008], [0011]-[0015] | 1-6 |

☒  Further documents are listed in the continuation of Box C.     ☒  See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 24 June 2021 (24.06.2021) | 13 July 2021 (13.07.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2021/018428

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2018-526378 A (TAGRA BIOTECHNOLOGIES LTD.) 13 September 2018 (2018-09-13) claims, paragraphs [0256], [0279], [0290], [0294], [0332]-[0341], [0374]-[0375] | 1, 4-6, 8<br>7 |
| Y | JP 2007-503293 A (DANISCO A/S) 22 February 2007 (2007-02-22) claims, paragraphs [0037], [0088] | 7 |
| A | JP 2018-501242 A (L'OREAL) 18 January 2018 (2018-01-18) | 1-8 |
| A | JP 2013-59550 A (KINDAI UNIVERSITY) 04 April 2013 (2013-04-04) | 1-8 |

Form PCT/ISA/210 (continuation of second sheet) (January 2015)

**INTERNATIONAL SEARCH REPORT**
Information on patent family members

| | | International application No. |
|---|---|---|
| | | PCT/JP2021/018428 |

| Patent Documents referred in the Report | Publication Date | Patent Family | Publication Date |
|---|---|---|---|
| JP 2014-221724 A | 27 Nov. 2014 | (Family: none) | |
| JP 11-269049 A | 05 Oct. 1999 | (Family: none) | |
| JP 2002-241211 A | 28 Aug. 2002 | (Family: none) | |
| JP 2018-526378 A | 13 Sep. 2018 | US 2018/0243718 A1 claims, paragraphs [0283], [0306], [0317], [0321], [0359]-[0368], [0400]-[0401] WO 2017/037716 A2 EP 3344220 A2 KR 10-2018-0049013 A CN 108135806 A | |
| JP 2007-503293 A | 22 Feb. 2007 | US 2007/0042184 A1 claims, paragraphs [0051], [0112]-[0121] WO 2005/018794 A1 EP 1663471 A1 CN 1925908 A | |
| JP 2018-501242 A | 18 Jan. 2018 | US 2017/0360661 A1 WO 2016/097641 A1 EP 3233023 A1 KR 10-2017-0097709 A CN 107205893 A | |
| JP 2013-59550 A | 04 Apr. 2013 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020088329 A
- JP 2019202962 A
- JP 2009161460 A
- WO 2019156116 A1

**Non-patent literature cited in the description**

- *The Future of Surfactants to 2022,* 2017
- Colloids and Surfaces A: Physicochemical and Engineering Aspects. 20 December 2013, vol. 439, 23-34
- *DETERGENT & COSMETICS,* January 2008, vol. 31 (1), 28
- *DETERGENT & COSMETICS,* August 2002, vol. 25 (4), 11
- *The bulletin of Okayama University of Science: Number 50, Appl 3-22,* 2014
- **TEZUKA.** *Carbonydr. Res.,* 1995, vol. 273, 83